# EUROPEAN PATENT APPLICATION

(11) **EP 0 600 422 A1**
(43) Date of publication of application: **08.06.1994**
(21) Application number: 93119226.4
(22) Date of filing: 29.11.1993
(51) Int. Cl.: A61K 9/48

(54) **Hard film composition for capsules**

(30) Priority: 30.11.1992 JP 319583/92
(71) Applicant: WARNER-LAMBERT COMPANY, Morris Plains New Jersey 07950 (US)
(72) Inventor: Kokubun, Toshio, Tokyo (JP); Ohnuki, Hiroshi, Yokohama City, Kanagawa (JP); Shimizu, Toyokazu, Sagamihara City, Kanagawa (JP)
(74) Representative: Mansmann, Ivo

(57) **Abstract**

A hard film composition for capsules, comprising a gelatin, and 3 to 10 % by weight of an ester of a fatty acid and glycerol or a polyglycerol added based on said gelatin. The capsules formed by the composition of the invention may not be embrittled even when the moisture in the capsule film is reduced by charging therein a hygroscopic drug, minimizing the liability of cracking or chipping, and thus preventing leakage of the drug contained therein to be caused by the damage of capsules.

## Description

The present invention relates to a hard film composition for capsules to be used for forming hard gelatin capsules and for band sealing such hard gelatin capsules.

Hard gelatin capsules are widely utilized in the field of pharmaceutical preparations due to the ease of preparation and administration. However, the conventional hard gelatin capsules suffer a problem that the capsule film loses flexibility to undergo cracking or chipping if they are packed with a hygroscopic agent such as of powder or granule, since the moisture contained in the capsule film is absorbed by the drug, particularly that the capsules are damaged when the capsules filled with a drug are packaged or they are taken out of the package for administration to let the drugs leak from the capsules.

Meanwhile, also known are hard film compositions for hard gelatin capsules having increased flexibility by adding a plasticizer such as glycerol, sorbitol and polyethylene glycol to a gelatin. However, such hard gelatin capsules suffer a problem in capsule manufacturing in that the capsule films become too soft and the drying speed thereof is retarded due to the plasticizer added.

Accordingly, under the present circumstances, the drugs to be contained in the conventional hard gelatin capsules are limited in order to prevent embrittlement of the capsule films to be caused by the moisture reduction.

It is an object of the present invention to provide a hard film composition for capsules which may not be embrittled even when the moisture in the capsule film is reduced by charging therein a hygroscopic drug, minimizing the liability of cracking or chipping and thus preventing leakage of the drug contained therein to be caused by the damage of the capsules.

The present invention is directed to a hard film composition for capsules, comprising gelatin, and 3 to 10 % by weight of an ester of a fatty acid and glycerol or a polyglycerol added based on said gelatin.

The present invention will be described below more specifically.

The ester of a fatty acid and glycerol to be employed according to the present invention can be exemplified by those of fatty acids of edible oil or fat origin and glycerol and derivatives thereof described in the Official Regulations on Food Additives. The fatty acids may not particularly be limited so long as they are of edible oil or fat origin and include, for example, saturated fatty acids such as palmitic acid and stearic acid or unsaturated fatty acids such as oleic acid and linoleic acid.

The ester of glycerol and a fatty acid, monoglyceride and a mixture of monoglyceride and diglyceride primarily containing monoglyceride (distilled monolglyceride) are preferred.

The ester of a fatty acid and glycerol also includes derivatives of such monoglyceride reacted with acetic acid, lactic acid, citric acid, succinic acid or diacetyltartaric acid.

Preferred of these esters of fatty acids and glycerol are glycerol monofatty acid esters, glycerol acetate fatty acid esters, glycerol lactate fatty acid esters, glycerol citrate fatty acid esters, glycerol succinate fatty acid esters, glycerol diacetyltartrate fatty acid esters and glycerol monoacetate.

Meanwhile, the ester of a fatty acid and a polyglycerol includes esters of a polyglycerol obtained by polymerization of 2 to 10 moles of glycerol and such fatty acids; and esters of a polyglycerol with a condensed ricinoleic acid obtained by condensation of 2 to 3 moles of ricinoleic acid, and preferred of them are polyglycerol monofatty acid esters and polyglycerol condensed ricinoleate.

The ester of a fatty acid and glycerol or a polyglycerol (hereinafter referred to as glycerol fatty acid ester) is preferably added in an amount of 3 to 10 % by weight, most preferably in an amount of 5 % by weight based on the gelatin. If the ester is added in an amount of less than 3 % by weight, brittleness of the resulting capsules may not be improved, whereas if the ester is added in an amount of more than 10 % by weight, the resulting molded capsules may sometimes be deformed, unfavorably.

As the process for preparing the hard film composition for capsules according to the present invention, any of the conventional methods can be utilized. For example, a capsule manufacturing composition is prepared by adding a predetermined amount of glycerol fatty acid ester to an aqueous gelatin solution, and the resulting composition is supplied to a capsule manufacturing machine to form capsules. Meanwhile, if a hydrophilic glycerol fatty acid ester is to be added to the agueous gelatin solution, it is preferably added after emulsification of the ester to an O/W type emulsion. Further, when the glycerol fatty acid ester is added to the gelatin, titanium oxide, a coloring agent such as dyes and other known additives can be added thereto.

### Example 1

A glycerol acetic acid fatty acid ester, warm water and an aqueous gelatin solution were mixed at a volume ratio of 2 : 3 : 5, and the resulting mixture was emulsified using a commercially available emulsifying machine to provide an O/W type dispersion. The dispersion was added to a 34 wt % aqueous gelatin solution prepared by dissolving a gelatin powder in a warm water such that the amount of the dispersion may be 5 wt % of the gelatin, and then a coloring agent, titanium oxide, etc. were added thereto, followed by viscosity adjustment to provide a capsule manufacturing composition.

The thus prepared composition was supplied to the capsule manufacturing machine to make hard capsules. The capsules thus obtained were subjected to empty capsule crack test, packed capsule pressurization test and disintegration test, and the test results are shown in Tables, 1, 2 and 3, respectively.

### Empty capsule crack test

The capsules obtained were tested using a Frank tester (manufactured by Carl Frank GmbH) by measuring the percentage (%) of cracked capsules in 50 capsules tested when the power at cracking was set to 0.5 J.

### Packed capsule pressurization test

The capsules obtained were packed with a corn starch, and a load of 5 kg was applied thereto to determine the proportion (%) of broken capsules in 50 capsules tested.

### Disintegration test

According to the Pharmacopoeia of Japan (Revision XII), the capsules packed with lactose were immersed in a distilled water at 37 ± 2°C to determine the time until the capsules were ruptured, the time until the contents were released and the time until the capsules were fully dissolved.

The above tests were also carried out for control capsules containing no glycerol fatty acid ester, and the test results are also shown in the respective Tables.

**Table 1**

| Empty Capsule Crack Test (Unit: %) | | | | |
|---|---|---|---|---|
| Capsule water content (%) | 13 % | 11 % | 9 % | 8 % |
| Capsule of the invention | 0 | 21 | 61 | 85 |
| Control capsule | 1 | 45 | 89 | 99 |

**Table 2**

| Packed Capsule Pressurization Test (Unit: %) | | | | |
|---|---|---|---|---|
| Capsule water content (%) | 13 % | 11 % | 9 % | 8 % |
| Capsule of the invention | 0 | 0 | 2.9 | 11.8 |
| Control capsule | 0 | 2.7 | 10.9 | 26.8 |

**Table 3**

| Disintegration Test (Unit: seconds) | | | |
|---|---|---|---|
| | Time until ruptured | Time until released | Time until dissolved |
| Capsule of invention | 53 | 110 | 672 |
| Control capsule | 46 | 108 | 756 |

As clearly shown in Tables 1 and 2, the capsules obtained using the composition of the present invention have excellent capsule strength, particularly in the low moisture range compared with the conventional capsules and the control capsules (containing polyethylene glycol only), whereas Table 3 shows that the present capsules have the same disintegrativity as those of the conventional capsules. Therefore, the present capsules agree with the Pharmacopoeia of Japan.

The capsules formed by using the hard film composition according to the present invention are not embrittled, even when the moisture in the capsule film is reduced by charging therein a hygroscopic agent, minimizing the liability of cracking or chipping, and thus preventing leakage of the drug contained therein to be caused by the damage of the capsules.

## Claims

1. A hard film composition for capsules, comprising a gelatin, and 3 to 10 % by weight based on said gelatin an ester of a fatty acid and glycerol or a polyglycerol which may be additionally esterified with aliphatic saturated or unsaturated mono- or dicarboxylic acids containing up to 10 carbon atoms.

2. A hard film composition for capsules according to claim 1, characterized in that the ester of a fatty acid and glycerol or a polyglycerol is contained in an amount of 5 % by weight.

3. A hard film composition for capsules according to claim 1 or 2, wherein the fatty acid esterified with glycerol to a mono- or diester is of edible oil or fat origin or hydrogenated products thereof, preferred palmitic acid, stearic acid, oleic acid, linoleic acid or ricinoleic acid.

4. A hard film composition for capsules according to claims 1 to 3, wherein the glycerol fatty acid ester is additionally esterified with acetic acid, lactic acid, citric acid, succinic acid or diacetyltartaric acid.

5. A hard film composition for capsules according to claims 1 to 3, wherein the glycerol fatty acid ester is glycerol monofatty acid esters, glycerol acetate fatty acid esters, glycerol lactate fatty acid esters, glycerol citrate fatty acid esters, glycerol succinate fatty acid esters, glycerol diacetyltartrate fatty acid esters and glycerol monoacetate.

6. A hard film composition for capsules according to claims 1 to 2, wherein the polyglycerol obtained by polymerization of 2 to 10 moles of glycerol is esterified with a fatty acid or a condensed ricinoleic acid, preferred polyglycerol monofatty acid ester and polyglycerol condensed ricinoleate.

7. Process for the production of hard gelatin capsules according to claims 1 to 6, comprising either mixing an aqueous emulsion of the glycerol or polyglycerol fatty acid ester with an aqueous gelatin solution or emulsifying the glycerol or polyglycerol fatty acid ester in an aqueous gelatin solution and thereafter dip moulding the capsule.
